# EUROPEAN PATENT APPLICATION

(11) **EP 4 775 200 A1**
(43) Date of publication of application: **15.07.2026**
(21) Application number: 25224632.7
(22) Date of filing: 17.12.2025
(51) Int. Cl.: A61K 9/00, A61K 9/10, A61K 47/14, A61K 47/26, A61K 47/38

(54) **IMMEDIATE-RELEASE, LIQUID ORAL PHARMACEUTICAL SUSPENSION DOSAGE FORM OF ESLICARBAZEPINE ACETATE**

(30) Priority: 14.01.2025 US 202519019671
(71) Applicant: Saptalis Pharmaceuticals, LLC, Hauppauge New York 11788 (US)
(72) Inventor: Kuznetsova, Larisa, Hauppauge, 11788 (US); Dondeti, Polireddy, Hauppauge, 11788 (US); Naringrekar, Amol, Hauppauge, 11788 (US)
(74) Representative: Turner, Craig Robert

(57) **Abstract**

An immediate-release liquid oral suspension of Eslicarbazepine Acetate, containing between about 5 to about 10 g of active ingredient per 100 mL, along with carbomer homopolymer and microcrystalline cellulose as viscosity modifying agents. It may further comprise, parabens as antimicrobial agent, sucralose as a sweetener, and polyethylene glycol 8 stearate as a surfactant. The suspension is designed to provide bioequivalence to marketed Eslicarbazepine Acetate tablets, ensuring rapid drug release and stability over extended storage periods. The pH is adjusted to 3.0-6.5 to maintain stability, with impurities remaining under 0.05% of the eslicarbazepine content after stress storage conditions. This formulation offers an alternative to solid dosage forms for patients with partial-onset seizures.

## Description

### FIELD OF THE INVENTION

The present invention relates to pharmaceutical formulations, specifically to an Immediate-release, liquid oral pharmaceutical suspension dosage form of Eslicarbazepine Acetate. The formulation is designed to treat partial-onset seizures, providing an alternative to tablet forms. The invention also covers methods for preparing the suspension and its use in medical treatments.

### BACKGROUND OF INVENTION

Eslicarbazepine acetate, chemically known as (S)-(-)-10-acetoxy-10,11-dihydro-5H-dibenz[b,f]azepine-5-carboxamide, a voltage-gated sodium channel (VGSC) blocker, is an anticonvulsant medication used primarily in the treatment of partial onset seizures. In the United States, it is available as immediate-release tablets marketed under the brand name APTIOM^{®} in strengths of 200 mg, 400 mg, 600 mg, and 800 mg by Sunovion Pharmaceuticals. While the solid dosage form is effective, its large size can pose challenges for many patients who have trouble swallowing (dysphagia). This issue is particularly pronounced among vulnerable populations, such as adolescents, children, and the elderly, who may struggle with large tablets or capsules. Such difficulties can lead to adverse events and contribute to non-compliance with prescribed treatment regimens.

To address these issues, liquid dosage forms have been developed to enhance patient compliance by minimizing swallowing difficulties. Liquid formulations are typically easier to administer, allowing for more flexible dosing adjustments and potentially improving adherence to treatment plans.

Previous formulations, such as those disclosed in U.S. Publication No. 2013/0040939 (BIAL), included oral suspensions of eslicarbazepine acetate using xanthan gum as a suspending agent, along with polyoxyethylene stearate as a wetting agent and saccharin sodium as a sweetener. However, the use of xanthan gum poses drawbacks, including susceptibility to microbial contamination, potential hard cake formation of the active suspended ingredient and the potential for adverse effects such as allergic reactions, bloating, and gastrointestinal discomfort. Additionally, sodium saccharin is associated with potential carcinogenic risks, further limiting its desirability as a sweetener.

Further, Jubilant patents disclose an eslicarbazepine suspension comprising eslicarbazepine acetate at concentrations ranging from about 0.1% to about 40% w/v, alongside suspending agents, surfactants, and a pharmaceutically acceptable liquid carrier. This formulation is designed to provide an optimal pH of 3 to 6 at buffer concentrations between about 5 mM to about 25 mM. Notably, the composition is free from certain suspending agents such as xanthan gum and carbomer and contains essential amount of sorbitol to enhance its organoleptic properties and ensuring stability. Although sorbitol solution improves palatability of the formulation, the excipient contains diethylene glycol and ethylene glycol as residual solvents, which should be avoided in paediatric formulations.

There is a clear need for stable liquid formulations of eslicarbazepine acetate that facilitate ease of administration, allow for dose adjustments, and enhance overall patient compliance. The inventors of the present invention have developed a ready-to-use eslicarbazepine suspension that addresses these challenges. These formulations are designed to be convenient for administration by paediatric and geriatric patients, easy to manufacture, palatable, functionally reproducible, and amenable to dose adjustments. Importantly, the liquid compositions exhibit desirable technical attributes that enhance their overall performance and usability.

### SUMMARY OF THE INVENTION

This invention provides an Immediate-release, liquid oral pharmaceutical suspension dosage form that includes: eslicarbazepine acetate as the active ingredient, viscosity modifying agent, surfactant, and may further have antimicrobials, pH adjusters, and flavoring agents or sweeteners. The composition, in the form of an aqueous suspension, contains about 8% eslicarbazepine acetate by weight, with specific proportions of other components.

The invention also covers the method of preparation, which involves dispersing or dissolving Eslicarbazepine Acetate in purified water and adjusting the pH with sodium hydroxide to form a homogeneous suspension. The formulation is designed for ease of use in patients who prefer or require liquid dosage forms.

The invention also outlines a method of administering eslicarbazepine acetate via oral suspension to patients and a process for preparing a stable suspension by mixing the active ingredient with a liquid carrier and incorporating other components such as viscosity modifying agents, surfactants, antimicrobial agents, pH adjusters, sweeteners and flavoring agents.

It further provides an immediate-release, liquid oral suspension dosage form of Eslicarbazepine Acetate for the treatment of partial-onset seizures. The suspension comprises Eslicarbazepine Acetate or its pharmaceutically acceptable salt, ester, hydrate, or polymorph, along with viscosity modifying agents, surfactants, antimicrobial agent, sweeteners, and flavoring agent, formulated to maintain stability, dissolution profile, and bioequivalence with marketed Eslicarbazepine Acetate tablets.

The suspension achieves over 75% drug release within 15 minutes, demonstrating fast onset of action, and remains stable under accelerated storage conditions. The viscosity of the suspension is optimized for ease of administration, and it is bioequivalent to tablet forms of Eslicarbazepine Acetate. The pH of the suspension is controlled within a range of 3.0 to 6.5, specifically 4.4-5.4 to ensure stability and minimize impurities.

### BRIEF DESCRIPTION OF THE FIGURES

Figure 1 is a graph of In Vitro Dissolution Results for Eslicarbazepine Acetate Oral Suspension, 400 mg/ 5 mL.
Figure 2 is a graph of Mean Plasma Eslicarbazepine Concentrations Vs Time on a linear scale of Aptiom 800 mg (R) and two test formulations according to the present disclosure (T1 and T2).

### DETAILED DESCRIPTION OF THE INVENTION

The present invention can be better understood through the following detailed description and accompanying examples.

In this context, the terms "composition," "formulation," and "dosage form" refer to a drug product containing an anticonvulsant or anti-epileptic agent, preferably eslicarbazepine or its pharmaceutically acceptable salts, esters, solvates, polymorphs, enantiomers, or mixtures thereof, combined with inert ingredients (pharmaceutically acceptable excipients). These terms are used interchangeably and cover various types of pharmaceutical compositions, including but not limited to suspensions that are ready-to-use.

The term " suspension" refers to liquid preparations that consist of solid particles dispersed throughout a liquid phase in which the particles are not soluble.

The term "ready-to-use suspension" refers specifically to a suspension that is pre-prepared and can be administered without further preparation.

The term "immediate release suspension" refers to a suspension wherein more than 75% drug is released within 15 minutes when determined using a USP type II apparatus at 100 rpm in 1000 mL of acetate buffer (pH 4.5) at 37 ± 0.5° C by an HPLC method.

The term "eslicarbazepine" broadly includes eslicarbazepine itself, eslicarbazepine acetate, as well as its pharmaceutically acceptable salts, solvates, hydrates, enantiomers, derivatives, isomers, polymorphs, metabolites, prodrugs, and its various crystalline and amorphous forms. Specifically, eslicarbazepine acetate is the S-isomer in a substantially pure form, with at least 98% purity.

The term "excipient" refers to pharmacologically inactive components, such as viscosity-modifying agents, suspending agents, surfactants, antimicrobial agents, anticaking agents, antifoaming agents, pH adjusters, antioxidants, sweeteners, flavoring agents, solubilizers, wetting agents, buffers, carriers, and similar substances. Excipients used in liquid pharmaceutical compositions are safe, non-toxic, and may include combinations of excipients or co-processed excipients to achieve desired formulation characteristics.

The term "about" indicates a range of ±20% around the specified value, meaning "about 10%" covers a range from approximately 8% to 12%.

In this specification, singular terms like "a," "an," and "the" are intended to include plural references unless explicitly stated otherwise. For example, a reference to "a process" includes one or more processes or steps, as described herein or as may become apparent to those skilled in the art.

The term q.s. an abbreviation for Quantum satis refers to the amount which is enough.

The term "Grams per 100 millilitres (g/100 mL)") refers to a concentration measure that represents the amount of a substance, expressed in grams, dissolved or mixed in 100 millilitres of the total solution. It is commonly referred to as weight/volume (w/v) concentration, also expressed as % w/v.

The term "stable" refers to chemical stability, where no more than 5% (w/v) of related substances are formed after storage at 40°C and 75% relative humidity (R.H.), or at 25°C and 60% R.H. for a minimum of one month, particularly for two months, and more preferably for at least three months.

The term "sedimentation volume ratio" or "sedimentation ratio" refers to the ratio of the final volume of sediment (Vu) to the original volume (VO) before settling, or the ratio of the ultimate height of sediment (Hu) to its initial height (HO) before settling.

The term "D50" represents the median particle size of a distribution, meaning that 50% of the particles (by volume or mass) are smaller than or equal to this size, while the other 50% are larger.

The term "D90" represents the particle size below which 90% of the particles (by volume or mass) are smaller, and 10% are larger.

"Taste-masking agents" refer to compounds that block taste receptors or mask undesirable taste properties (e.g., chalkiness, grittiness, dryness, bitterness) of an active ingredient.

The term "administering" or "administration" refers to the physical introduction of a composition comprising a therapeutic agent to a subject, using any of the various methods and delivery systems known to those skilled in the art. Administering can be performed, for example, once, multiple times, and/or over extended periods and can involve a therapeutically effective dose or a subtherapeutic dose.

The term "ease of administration" refers to the ability of a pharmaceutical oral suspension to be accurately measured, conveniently consumed, and readily handled by patients or caregivers, ensuring consistent dosing, palatability, and suitability for the intended patient population.

The term "bioequivalence" refers to an immediate release oral pharmaceutical suspension of present invention, wherein the suspension is bioequivalent to marketed Eslicarbazepine Acetate Oral Tablets 200 mg, 400 mg, 600 mg, and 800 mg, based on:
(a) pharmacokinetic parameters (Cmax, AUC0-t, and AUC0-∞) with geometric mean ratios and 90% confidence intervals within the bioequivalence range of 80-125%; (b) evaluation in a randomized crossover study under fasting conditions with a 7-day washout; and (c) comparable bioavailability and tolerability to Aptiom^{®} Eslicarbazepine Acetate Tablets.

The term "impurities" refers to specified or unspecified degradation products present in the pharmaceutical suspension of Eslicarbazepine, wherein specified degradation products include oxcarbazepine (NMT 0.15%), Carbamazepine (NMT 0.15%), and any unspecified degradation product (NMT 0.10%), with the total impurities not exceeding 2.0% of the eslicarbazepine content.

Unless otherwise stated, weight percentages mentioned herein are based on the final weight of the composition or formulation.

The present invention aims to provide a stable, Immediate-release, liquid oral pharmaceutical suspension dosage form containing an anticonvulsant drug, alongside one or more pharmaceutically acceptable excipients, and processes for its preparation.

The invention further provides an Immediate-release, liquid oral pharmaceutical suspension dosage form comprising eslicarbazepine or its pharmaceutically acceptable esters, salts, solvates, polymorphs, enantiomers, or mixtures thereof, along with one or more pharmaceutically acceptable excipients and/or carriers, and processes for their preparation.

Additionally, the invention provides a suspension and a suspension powder for reconstitution, comprising eslicarbazepine or its pharmaceutically acceptable esters, salts, solvates, polymorphs, enantiomers, or mixtures thereof, combined with one or more pharmaceutically acceptable excipients and/or carriers, and processes for their preparation.

The present invention also covers an oral liquid ready to use pharmaceutical composition in suspension form, comprising eslicarbazepine or its pharmaceutically acceptable esters, salts, solvates, polymorphs, enantiomers, or mixtures thereof, and one or more pharmaceutically acceptable excipients selected from the group consisting of viscosity-modifying agents, antioxidants, anticaking agents, antifoaming agents, pH-adjusting agents, coloring agents, sweetening agents, flavoring agents, surfactants, solubilizers, wetting agents, buffers, diluents, antimicrobial agents, and combinations thereof. The suspension may be either a ready-to-use suspension or a suspension powder for reconstitution.

The present invention provides an Immediate-release, liquid oral pharmaceutical suspension dosage form comprising eslicarbazepine acetate, a liquid carrier component, and one or more pharmaceutically acceptable excipients.

For example, provided herein is an immediate-release, liquid oral pharmaceutical suspension dosage form comprises:
(a) Eslicarbazepine Acetate, or a pharmaceutically acceptable salt, ester, hydrate, or polymorph thereof;
(b) Carbomer;
(c) Microcrystalline cellulose;
(d) optionally Polyethylene Glycol 8 Stearate, an antimicrobial agent, a sweetening agent, a flavoring agent, or any combination thereof;
(e) Purified water, as the carrier, q.s. to 100 mL;
(f) one or more buffers or pH adjusting agents, present in an amount effective to provide the suspension with a pH of from about 3.0 to about 6.5.

In addition to the components expressly described, the pharmaceutical suspension may comprise other pharmaceutically acceptable excipients known in the art. For example, it may include one or more additional antimicrobial agents, antioxidants, colorants, stabilizers, surfactants, taste-masking agents, viscosity modifiers, flavoring agents, or combinations thereof, without departing from the scope of the present disclosure.

The Immediate-release, liquid oral pharmaceutical suspension dosage form may comprise an active ingredient component containing eslicarbazepine acetate. The suspension may include eslicarbazepine acetate in a concentration ranging from about 5% by weight to about 10% by weight of the suspension. In preferred embodiments, the suspension comprises eslicarbazepine acetate at about 8% by weight.

The suspension dosage form may also include a liquid carrier component. The carrier, vehicle, or solvent used in the suspension can be aqueous or non-aqueous, such as water, alcohol, polyethylene glycol, propylene glycol, glycerin, buffers, oil, or combinations thereof. Oils may include peanut oil, soybean oil, corn oil, sesame oil, cottonseed oil, acetylated glycerides, ethyl oleate, mineral oil, fatty acid esters, mono- or di-fatty acid esters of polyethylene glycols, or glyceryl mono-oleate. The suspensions are particularly aqueous-based, meaning they consist of water or a combination of water and a water-miscible organic solvent(s) such as propylene glycol, polyethylene glycol, glycerin, sorbitol, or ethanol. "Non-aqueous carrier" refers to a suspension where the carrier does not contain water. Additionally, the carrier may include one or more pharmaceutically acceptable excipients in dissolved or dispersed form and is specifically water or a combination of water and glycerin.

Water may generally account for the balance of the suspension, in combination with other components discussed herein. For example, the suspension may contain water in a concentration of about 75% to 95% w/v, with specific ranges of 10%-99%, 30%-95%, 40%-95%, 50%-95%, and 60%-95% w/v.

The suspension dosage form may also include at least one viscosity modifying agent, which improves the physical stability of the suspension by increasing viscosity to slow settling, while maintaining adequate pourability. These agents also ensure that the product is easily resuspendable, allowing accurate dosing with minimal shaking. Suitable agents include cellulose derivatives such as hydroxyethyl cellulose, hydroxypropyl cellulose, hydroxypropylmethyl cellulose, methylcellulose, carboxymethyl cellulose and its salts (e.g., carboxymethyl cellulose sodium), microcrystalline cellulose, co-processed spray-dried forms of microcrystalline cellulose and carboxymethyl cellulose sodium (e.g., AVICEL^{®} RC-501, RC-581, RC-591, CL-611), carbomers (e.g., CARBOPOL^{®}), and various gums (locust bean, tragacanth, arabinogalactan, agar, gellan, guar, apricot, karaya, sterculia, acacia, gum arabic, carrageenan), among others. The preferred viscosity modifying agent is carbomer homopolymer, either alone or in combination with microcrystalline cellulose. A non-limiting example of a commercially available carbomer homopolymer is carbomer homopolymer Type B (Carbopol 974P). A non-limiting example of a commercially available microcrystalline cellulose is Avicel PH101. These agents are present in amounts ranging from 0.01% to 20% w/v, particularly 0.01%-10.0%, 0.01%-5.0%, 0.01%-4.0%, 0.01%-3.0%, or 0.01%-2.0% w/v.

The suspension is easily pourable and, upon shaking, exhibits a viscosity range of 30 to 850 cPs at 25°C, with specific ranges of 100-850 cPs. "Shaken" refers to vigorous shaking before use (e.g., by hand for 5 to 40 seconds). Viscosity can be measured using instruments like Brookfield viscometers or Haake VT 550 viscometers at 25°C. The viscosity remains between 100-850 cPs when stored for up to one month at 40°C/75% R.H., measured using a Brookfield viscometer with spindle LV-3 at 100 rpm and 25°C.

The suspension dosage form may comprise a surfactant or emulsifier. Surfactants or wetting agents aid in dispersing eslicarbazepine and improving wettability. The amount of surfactant or wetting agent should be sufficient to facilitate the dispersion of eslicarbazepine in the suspension and improve its wettability. Suitable surfactants include non-ionic, anionic, cationic, or zwitterionic types and combinations thereof. Examples of wetting agents include sodium lauryl sulphate, cetrimide, polyethylene glycols, polyoxyethylene glycol esters (e.g., polyoxyethylene glycol 400 monostearate), polyglycerin fatty acid esters (e.g., decaglyceryl monolaurate, decaglyceryl monomyristate), sorbitan fatty acid esters (e.g., sorbitan monostearate), polyoxyethylene sorbitan fatty acid esters (e.g., polyoxyethylene sorbitan monooleate), polyoxyethylene alkyl ether (e.g., polyoxyethylene lauryl ether), polyoxyethylene castor oil, and polyoxyethylene-polyoxypropylene block copolymers (e.g., Poloxamer 188), among others. Surfactants are present in amounts from 0.01% to 3% w/v, particularly 0.1% to 3% w/v.

The suspension may also include an antimicrobial agent to ensure product stability. Examples include methylparaben, propylparaben, butylparaben, benzoic acid and its salts (e.g., sodium benzoate, potassium benzoate, calcium benzoate), para-hydroxybenzoates, sorbic acid and its salts (e.g., sodium sorbate, potassium sorbate), sodium metabisulfite, and chlorhexidine, among others. A particularly preferred antimicrobial component comprises a mixture of methylparaben and propylparaben.

The antimicrobial component may be present in a concentration of about 0.01% by weight to 2% by weight, with a specific range of 0.01% to 0.3% by weight.

Finally, the suspension may include a bulking agent to provide bulk. Suitable examples include sucrose, sugar alcohols (e.g., glycerin, sorbitol, xylitol, erythritol), starch, calcium carbonate, calcium phosphate (dibasic anhydrous, dibasic dihydrate, tribasic), cellulose (powdered, microcrystalline, silicified), maltodextrin, lactose, or combination thereof in a concentration of about 0.1% to 20% w/v.

The suspension may comprise an anticaking agent. Anticaking agents improve re-suspendability and may include colloidal silica, calcium phosphate tribasic, magnesium oxide, and others. These agents are present in amounts ranging from 0.1% to 10% w/v, particularly 0.5% to 7% w/v.

The pharmaceutical suspension may also include an antioxidant. Examples of antioxidants, such as ascorbic acid, tert-butylhydroquinone, sodium pyrosulfite, tocopherol, and others, are present in amounts of up to 10% w/v.

Additionally, the pharmaceutical suspension may comprise a sweetening agent. Sweetening agents may include sugars, sugar alcohols, or sugar substitutes. Examples include sugars such as sucrose, dextrose, fructose, lactose, maltose, and invert sugar; sugar alcohols like sorbitol, mannitol, xylitol, lactitol, erythritol, maltodextrin, maltitol, isomaltitol, isomalt, maltulose, isomaltulose, lactulose, threitol, arabitol, ribitol, and galactitol; and sugar substitutes like saccharin sodium, aspartame, thaumatin, acesulfame, or combinations thereof. Sugars and sugar alcohols can also act as fillers. Preferred sweetening agents include sodium saccharin, sucrose, dextrose, lactose, sorbitol, mannitol, sucralose, and sodium saccharin, with sucralose being the most preferred. Sweetening agents are present in amounts ranging from 0.01% to 99% w/v, with specific ranges of 0.01%-90%, 0.1%-80%, 0.1%-70%, 0.1%-60%, 0.1%-50%, 0.1%-40%, 0.1%-30%, 0.1%-20%, 0.1%-10%, and 0.1%-5% w/v.

The pharmaceutical suspension may also comprise a flavoring agent. Flavoring agents may include natural or synthetic flavours such as banana, lemon, orange, grape, lime, grapefruit, vanilla, bubble gum, peppermint, fantasy fruit masking flavour, and fruit essences including apple, banana, pear, peach, strawberry, raspberry, cherry, plum, pineapple, and apricot. Other examples include synthetic flavour oils, flavoring aromatics, or natural oils like cinnamon oil, oil of wintergreen, peppermint oil, clove oil, citrus oil, bay oil, anise oil, eucalyptus, thyme oil, cedar leaf oil, oil of nutmeg, oil of sage, oil of bitter almonds, cassia oil, maltol, ethyl vanillin, menthol, citric acid, fumaric acid, ethyl maltol, tartaric acid, and combinations thereof. Flavoring agents are present in amounts of 0.01% to 7% w/v, particularly 0.01% to 20%, 0.01% to 15%, 0.01% to 10%, 0.01% to 5%, 0.01% to 3%, or 0.01% to 1% w/v.

The pharmaceutical suspension may comprise an isotonicity-adjusting agent. Examples include sodium chloride, mannitol, sorbitol, glucose, and glycerin.

Finally, the pharmaceutical suspension may comprise pH-adjusting or buffering agents. These may include, but are not limited to, citrate buffers, phosphate buffers, or other suitable buffers known in the art, such as monosodium dibasic phosphate, gluconic acid, lactic acid, citric acid, trisodium citrate, acetic acid, maleic acid, tartaric acid, fumaric acid, sodium phosphate, sodium gluconate, sodium lactate, sodium citrate, sodium acetate, potassium citrate, sodium bicarbonate, potassium bicarbonate, sodium dihydrogen phosphate, and potassium dihydrogen phosphate. They may also include combinations of acidic and basic substances. Strong acids such as hydrochloric acid, sulfuric acid, phosphoric acid, and the like can be used alone or in combination with basic substances like inorganic bases (e.g., sodium hydroxide, potassium hydroxide, sodium carbonate, sodium hydrogen carbonate, magnesium carbonate, calcium carbonate, magnesium oxide, ammonia, synthetic hydrotalcite), or organic bases (e.g., basic amino acids such as lysine, arginine, meglumine, and others). Examples of buffers include citrate and phosphate buffers or sodium hydroxide, among others known in the art.

The pharmaceutical suspension may be provided in a dosage form comprising a therapeutically effective amount of eslicarbazepine acetate. For instance, the dosage form may contain at least about 0.1 mg, 1 mg, 10 mg, 20 mg, 30 mg, 40 mg, 50 mg, 60 mg, 70 mg, or 80 mg per ml of eslicarbazepine acetate. Conversely, the dosage form may contain at most about 1000 mg, 800 mg, 600 mg, 400 mg, 200 mg, 150 mg, 100 mg, 90 mg, or 80 mg per 100 ml of eslicarbazepine acetate. Non-limiting examples of therapeutically effective amounts include about 0.1 mg, 1 mg, 10 mg, 37.5 mg, 75 mg, 100 mg, 200 mg, 400 mg, 600 mg, 800 mg, or 1000 mg per 100 mL.

The pharmaceutical suspension may comprise eslicarbazepine acetate in amounts within ranges defined by any of the values listed above. For example, it may contain from about 10 mg to about 1000 mg, 25 mg to 800 mg, 25 mg to 600 mg, 25 mg to 400 mg, or 50 mg to 200 mg per 100 mL.

The pharmaceutical suspension may be provided in the form of a liquid suspension of eslicarbazepine acetate suitable for oral administration. In one embodiment, a suspension contains eslicarbazepine acetate (0.1% to 10% w/v), a viscosity-modifying agent (0.01% to 3% w/v), and a surfactant (0.01% to 3% w/v), which exhibits more than 75% drug release within 15 minutes under specified dissolution conditions. The invention also covers both ready-to-use suspensions and suspension powders for reconstitution of eslicarbazepine.

The suspension dosage form may exhibit a viscosity range from about 30 cps to about 850 cps. Another embodiment provides a suspension comprising eslicarbazepine, a viscosity-modifying agent, a surfactant, an antimicrobial agent, optionally an antioxidant, and a pH-adjusting agent to maintain a pH between 3.0 and 6.5, along with a liquid carrier.

Another suspension embodiment includes eslicarbazepine, a viscosity-modifying agent selected from alginates, methylcellulose, hydroxyethyl cellulose, carboxymethylcellulose, sodium carboxymethylcellulose, microcrystalline cellulose, acacia, tragacanth, bentonite, carbomer, carrageenan, powdered cellulose, and gelatine; a surfactant; a preservative; and optionally an antioxidant. The selection of the viscosity-modifying agent depends on other ingredients that contribute viscosity to the medium. The stability of suspensions depends more on the types of viscosity-modifying agents than on the physical properties of the drugs.

Another embodiment includes eslicarbazepine, a viscosity-modifying agent, and a surfactant selected from non-ionic, anionic, cationic, or zwitterionic surfactants; a preservative; and optionally an antioxidant up to 10% w/v of the suspension.

In yet another embodiment, the suspension contains eslicarbazepine acetate (5 g/100 mL to 10 g/100 mL), has a pH between 3.0 and 6.5, and a viscosity ranging from 30 cps to 850 cps.

Another embodiment includes eslicarbazepine acetate at a concentration of about 5 g to about 10 g per 100 mL; carbomer as a stabilizer and viscosity-modifying agent at about 0.01 g to about 1.0 g per 100 mL; microcrystalline cellulose as a viscosity-modifying agent at about 0.1 g to about 3.0 g per 100 mL; polyethylene glycol 8 stearate to enhance drug wettability at about 0.01 g to about 3.0 g per 100 mL; methylparaben and propylparaben as antimicrobial agents in amounts ranging from about 0.01 g to about 0.30 g per 100 mL; sucralose as a non-caloric sweetener at about 0.1 g to 1.0 g per 100 mL; and purified water as the primary vehicle.

In another embodiment, the suspension contains approximately 8 g of eslicarbazepine acetate per 100 mL, carbomer at about 0.01 g to 1.0 g per 100 mL, microcrystalline cellulose at about 0.1 g to 3.0 g per 100 mL, polyethylene glycol 8 stearate at about 0.01 g to 3.0 g per 100 mL, methylparaben (0.12 g), propylparaben (0.03 g), and sucralose (0.3 g).

In yet another embodiment, an oral suspension comprises 8 g of Eslicarbazepine Acetate per 100 mL, along with 0.1 g of carbomer, 0.6 g of microcrystalline cellulose, 0.12 g of methylparaben, 0.03 g of propylparaben, 0.3 g of sucralose, and 0.1 g of polyethylene glycol 8 stearate, with the pH adjusted to 4.9.

In yet another embodiment, an alternative suspension contains an Eslicarbazepine Acetate concentration of 10 g per 100 mL, 0.1 g of carbomer, 0.6 g of microcrystalline cellulose, 0.12 g of methylparaben, 0.03 g of propylparaben, 0.3 g of sucralose, and 0.1 g of polyethylene glycol 8 stearate, with the pH adjusted to 4.9.

In yet another embodiment, the suspension consists of Eslicarbazepine Acetate in an amount ranging from about 5 g to 10 g per 100 mL of suspension, with variants that may include pharmaceutically acceptable salts, esters, hydrates, or polymorphs. The suspension also includes carbomer homopolymer and microcrystalline cellulose as viscosity-modifying agents, which contribute to its viscosity and stability. To ensure product stability and safety, at least one paraben preservative, such as methylparaben or propylparaben, is incorporated. Sucralose, NF, is used to provide a pleasant taste, and polyethylene glycol 8 stearate serves as a surfactant to aid in the dispersion of the active pharmaceutical ingredient (API) and other components. Purified water, acts as the carrier to form the suspension, while sodium hydroxide is used to adjust the pH to a range of about 4.4 to 5.4, optimizing the solubility and/or stability of Eslicarbazepine Acetate.

In another embodiment the oral pharmaceutical suspension dosage form further comprises:
- at least one paraben preservative;
- a sweetener;
- a flavoring agent.

In another embodiment the oral pharmaceutical suspension dosage form comprises:
(a) Eslicarbazepine Acetate, or a pharmaceutically acceptable salt, ester, hydrate, or polymorph thereof, in an amount from about 5 to about 10 g per 100 mL of the suspension;
(b) Carbomer, in an amount ranging from about 0.01 g to about 1.0 g per 100 mL of the suspension;
(c) Microcrystalline cellulose, in an amount ranging from about 0.1 g to about 3.0 g per 100 mL of the suspension;
(d) Polyethylene Glycol 8 Stearate, in an amount ranging from about 0.01 g to about 3.0 g per 100 mL of the suspension;
(e) at least one paraben preservative in an amount ranging from about 0.01 g to about 0.30 g per 100 mL of the suspension;
(f) Sucralose, in an amount ranging from about 0.10 g to about 1.0 g per 100 mL of the suspension;
(g) a flavoring agent in an amount ranging from about 0.1 g to about 1.0 g per 100 mL of the suspension;
(h) Purified water, as the carrier, q.s. to 100 mL;
(i) one or more buffers or pH adjusting agents selected from citrate buffers, phosphate buffers, or sodium hydroxide, present in an amount effective to provide the suspension with a pH of from about 3.0 to about 6.5.

In yet another embodiment the oral pharmaceutical suspension dosage form comprises:
(a) Eslicarbazepine Acetate, or a pharmaceutically acceptable salt, ester, hydrate, or polymorph thereof, in an amount of about 8.0 g per 100 mL of the suspension;
(b) Carbomer, in an amount from about 0.05 g to about 0.15 g per 100 mL;
(c) Microcrystalline cellulose, in an amount from about 0.3 g to about 1.0 g per 100 mL;
(d) Polyethylene Glycol 8 Stearate, in an amount of about 0.1 g per 100 mL;
(e) Methylparaben, in an amount of about 0.12 g per 100 mL;
(f) Propylparaben, in an amount of about 0.03 g per 100 mL;
(g) Sucralose, in an amount of about 0.30 g per 100 mL;
(h) Natural Peppermint Flavor, in an amount of about 0.30 g per 100 mL;
(i) Sodium hydroxide for pH adjustment; and
(j) Purified water, as the carrier, q.s. to 100 mL;
wherein the pH of the suspension is from about 4.4 to about 5.4.

A suspension comprises eslicarbazepine and one or more pharmaceutically acceptable excipients. These excipients may include viscosity-modifying agents, antioxidants, anticaking agents, antifoaming agents, pH-adjusting agents, colouring agents, sweeteners, flavoring agents, surfactants, buffers, diluents, and antimicrobial agents.

A stable, ready-to-use liquid suspension of eslicarbazepine is also provided, including excipients such as viscosity-modifying agents, antioxidants, anticaking agents, antifoaming agents, pH-adjusting agents, colouring agents, sweeteners, flavoring agents, surfactants, buffers, diluents, taste-masking agents, and antimicrobial agents.

The invention includes a composition for use as a liquid suspension, suitable for children or elderly patients, comprising eslicarbazepine and excipients like viscosity-modifying agents, antimicrobial agents, flavoring agents, sweeteners, and lubricants.

The invention provides a process for preparing a stable pharmaceutical suspension containing eslicarbazepine or its pharmaceutically acceptable salts, esters, hydrates, or polymorphs.

In another embodiment, the preparation method involves: The preservative phase (A) is prepared by heating water to 80-85°C, then mixing in the antimicrobial agent. After cooling the mixture to 40-45°C, viscosity-modifying agents are dispersed, followed by the addition of buffering agents and sweeteners. The API phase (B) is created by heating 0.5 to 50 g of purified water to 45-55°C, mixing in the surfactant, and then cooling to 25-30°C before introducing the API. The resulting slurry is then transferred to phase (A), which has been cooled to 25-30°C. The combined mixture is vigorously stirred to form the bulk oral suspension.

In another embodiment a method of preparing an oral pharmaceutical suspension dosage form, comprises:
(a) Preparing a preservative phase (A) by: - Heating purified water to a temperature of 80-85°C; - Adding methylparaben and propylparaben to the heated water and mixing until dissolved; - Cooling the mixture to 40-45°C and dispersing carbomer homopolymer and microcrystalline cellulose into the mixture; - Adding buffering agents and sweeteners to the mixture and further cooling to 25-30°C;
(b) Preparing an API phase (B) by: - Heating 0.5 to 50 g of purified water to a temperature of 45-55°C; - Mixing polyethylene glycol 400 monostearate into the heated water and cooling the mixture to 25-30°C; - Adding Eslicarbazepine Acetate to form a slurry;
(c) Combining the API phase (B) with the preservative phase (A), wherein phase (B) is added to phase (A) at a temperature of 25-30°C;
(d) Adjusting the pH of the combined mixture (A and B) to between about 4.4 and 5.4 using sodium hydroxide; and
(e) Vigorously mixing the resulting combined mixture to form a homogeneous suspension.

Another embodiment involves a process for preparing a suspension in which eslicarbazepine is in micronized form. A process is also provided for preparing a ready-to-use liquid suspension using conventional equipment like overhead stirrers, ultrasonifiers, mills, and homogenizers.

Another embodiment provides a stable suspension of eslicarbazepine, free from polymorphic forms. The suspension formulation includes eslicarbazepine with a particle size distribution D90 particle size less than 250 µm and D50 particle size less than 150 µm.

Particle size measurements for determining D50 (median particle size) and D90 (particle size below which 90% of particles fall) are typically performed using techniques such as laser diffraction (LD), which measures the scattering of light by particles in a dispersed sample to generate a particle size distribution curve. Other methods include dynamic light scattering (DLS), which uses fluctuations in scattered light from particles undergoing Brownian motion to estimate size, and sieving, where particles are mechanically separated through a series of meshes with decreasing pore sizes to calculate cumulative size distributions. Sedimentation techniques, which analyze the settling velocity of particles in a fluid under gravity or centrifugal force, and microscopy methods such as scanning electron microscopy (SEM) or transmission electron microscopy (TEM), allow direct measurement of individual particles and detailed statistical analysis. The choice of method depends on the particle size range, material properties, and required precision.

The stable suspension formulation exhibits technical attributes such as pourability, viscosity, dissolution, stability, re-suspendability, and re-dispersibility.

Another embodiment provides a stable suspension of eslicarbazepine having long-term stability with less than 2% total impurities of the eslicarbazepine content.

In another embodiment, the viscosity of the suspension ranges from 30 cps to 850 cps, ensuring smooth flow and ease of administration, even when stored for up to one month under accelerated conditions.

Another embodiment provides a suspension of eslicarbazepine having viscosity from 30 cps to 850 cps as measured by a Brookfield viscometer using spindle LV-3 at 100 rpm and 25°C.

Another embodiment provides a suspension of eslicarbazepine having viscosity tailored to range from 100 cps to 300 cps for ease of administration to paediatric patients.

Another embodiment provides a suspension of eslicarbazepine wherein the suspension exhibits more than 75% of drug release within 15 minutes when placed in a dissolution vessel filled with 1000 mL of acetate buffer, pH 4.5, maintained at 37 ± 0.5°C and stirred at a paddle speed of 100 rpm using a USP Type II (paddle) apparatus.

Another embodiment provides a suspension of eslicarbazepine, wherein the suspension is bioequivalent to marketed Eslicarbazepine Acetate Oral Tablets (200 mg, 400 mg, 600 mg, and 800 mg) based on pharmacokinetic parameters (Cmax, AUC0-t, and AUC0-∞) with geometric mean ratios and 90% confidence intervals within the bioequivalence range of 80-125%.

In another embodiment, the suspension demonstrates bioequivalence to marketed Eslicarbazepine Acetate tablets (200 mg, 400 mg, 600 mg, and 800 mg) based on clinical studies, ensuring similar therapeutic outcomes when switching from tablet to liquid form.

Another embodiment provides a suspension of eslicarbazepine, wherein the pH of the suspension is about 4.9, and after storage at 60°C for 1-week, unknown impurities are less than 0.05% of the total eslicarbazepine content.

Another embodiment provides a suspension of eslicarbazepine, wherein the pH of the suspension is about 5.4, and after storage at 60°C for 1-week unknown impurities are less than 0.03% of the total eslicarbazepine content.

Another embodiment provides a suspension of eslicarbazepine, wherein the suspension maintains chemical stability and exhibits less than 0.5% w/v degradation of Eslicarbazepine Acetate after storage at 40°C and 75% relative humidity for six months.

The suspension formulation is also intended for treating conditions like epilepsy, neuropathic pain, migraine, fibromyalgia, bipolar disorders, and more.

In an embodiment, the immediate-release liquid oral pharmaceutical suspension of the present invention contains Eslicarbazepine Acetate as the active pharmaceutical ingredient (API), present in an amount ranging from approximately 5 g to 10 g per 100 mL of suspension. The API may include pharmaceutically acceptable salts, esters, hydrates, or polymorphs. The suspension uses carbomer homopolymer and microcrystalline cellulose as viscosity-modifying agents, enhancing its viscosity and stability. To maintain product stability and safety, at least one paraben preservative, such as methylparaben and propylparaben, is incorporated. Sucralose, is added as a sweetener to provide a pleasant taste. Polyethylene Glycol 8 Stearate serves as a surfactant, aiding in the dispersion of the API and other components. Purified water, is used as the carrier to form the suspension, and sodium hydroxide adjusts the pH to a range of about 4.4 to 5.4, optimizing the solubility and/or stability of Eslicarbazepine Acetate.

In another embodiment, the formulation is designed to ensure polymorphic stability, with no observed change in the polymorphic form of Eslicarbazepine Acetate when stored at 80°C for at least five days. Additionally, the suspension remains stable when stored at 40°C and 75% relative humidity for at least one month, demonstrating resilience under challenging storage conditions. The viscosity of the suspension is maintained within specific ranges to ensure ease of administration while preserving its essential suspension characteristics. The release profile of the suspension is rapid, with more than 75% of the active ingredient released within 15 minutes, ensuring an immediate therapeutic effect. Furthermore, the formulation is bioequivalent to marketed Eslicarbazepine Acetate oral tablets, providing confidence in its therapeutic efficacy and safety profile.

In another embodiment liquid oral pharmaceutical suspension dosage form is used for treating partial onset seizures in a patient in need thereof.

The following examples illustrate various aspects of the present invention. The examples should, of course, be understood to be merely illustrative of only certain embodiments of the invention and not to constitute limitations upon the scope of the invention, which is defined by the claims appended at the end of this description.

Having described the disclosure in detail, it will be apparent that modifications and variations are possible without departing from the scope of the claims.

### EXAMPLES

### Example 1

| **S. No** | **Ingredients** | **Formula Quantity (g/100mL)** |
|---|---|---|
| 1 | Eslicarbazepine Acetate | 8.00 g |
| 2 | Methylparaben, NF | 0.12 g |
| 3 | Propylparaben, NF | 0.03 g |
| 4 | Carbomer Homopolymer Type B, NF (Carbopol 974P, NF) | 0.05 g |
| 5 | Microcrystalline Cellulose, NF (Avicel PH101) | 0.60 g |
| 6 | PEG 8 stearate (Propylene Glycol 400 monostearate) | 0.10 g |
| 7 | Sucralose, NF | 0.30 g |
| 8 | Sodium Hydroxide, NF | 0.01 g |
| 9 | Natural Peppermint Flavor, 501500T | 0.30 g |
| 10 | Purified Water, USP | Q.S. to 100 mL |

### Example 2

| **S. No** | **Ingredients** | **Formula Quantity (g/100mL)** |
|---|---|---|
| 1 | Eslicarbazepine Acetate | 8.00 g |
| 2 | Methylparaben, NF | 0.12 g |
| 3 | Propylparaben, NF | 0.03 g |
| 4 | Carbomer Homopolymer Type B, NF (Carbopol 974P, NF) | 0.10 g |
| 5 | Microcrystalline Cellulose, NF (Avicel PH101) | 0.60 g |
| 6 | PEG 8 stearate (Propylene Glycol 400 monostearate) | 0.10 g |
| 7 | Sucralose Micronized Powder, NF (Splenda^{®}) | 0.30 g |
| 8 | Sodium Hydroxide, NF | 0.005 g |
| 9 | Natural Peppermint Flavor, 501500T | 0.30 g |
| 10 | Purified Water, USP | Q.S. to 100 mL |

### Example 3

| **S. No** | **Ingredients** | **Formula Quantity (g/100mL)** |
|---|---|---|
| **1** | **Eslicarbazepine Acetate** | **8.00 g** |
| 2 | Methylparaben, NF | 0.12 g |
| 3 | Propylparaben, NF | 0.03 g |
| 4 | Carbomer Homopolymer Type B, NF (Carbopol 974P, NF) | 0.12 g |
| 5 | Microcrystalline Cellulose, NF (Avicel PH101) | 0.60 g |
| 6 | PEG 8 stearate (Propylene Glycol 400 monostearate) | 0.10 g |
| 7 | Sucralose Micronized Powder, NF (Splenda^{®}) | 0.30 g |
| 8 | Hydrochloric Acid, 37% NF | 0.025 g |
| 9 | Natural Peppermint Flavor, 501500T | 0.30 g |
| 10 | Purified Water, USP | Q.S. to 100 mL |

### Method of preparation (Examples 1-3)

A preservative phase (A) is prepared by heating water to 80-85°C, then mixing in methylparaben and propylparaben. The mixture is cooled to 40-45°C, after which Carbopol 974P and microcrystalline cellulose are dispersed. Buffering agents and sweeteners are then added. Separately, an API phase (B) is prepared by heating 0.5 to 50 g of purified water to 45-55°C, mixing in polyethylene glycol 400 monostearate, and cooling the mixture to 25-30°C before introducing the API. The resulting slurry is then transferred to phase (A), which has been cooled to 25-30°C. The final mixture is vigorously stirred to form the bulk oral suspension.

### Dissolution Study

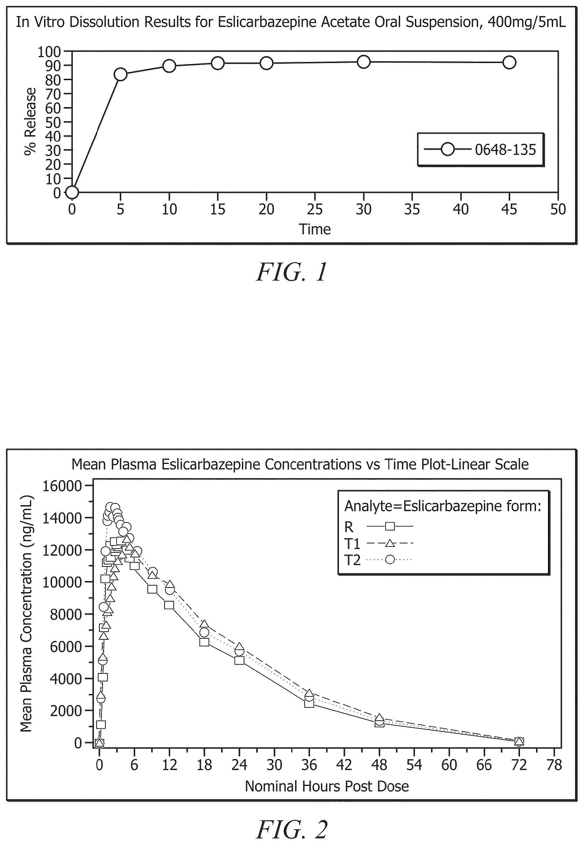

A study was conducted to determine the dissolution profile of an Eslicarbazepine Acetate Oral Suspension prepared as described above in Example 2. The eslicarbazepine suspension was evaluated for in vitro eslicarbazepine release. The in vitro dissolution was determined using a USP type II apparatus at 100 rpm in 1000 mL of acetate buffer (pH 4.5) at 37 ± 0.5° C by an HPLC method. The dissolution data are presented in Table 1A below. Figure 1 is a graph of In Vitro Dissolution Results for Eslicarbazepine Acetate Oral Suspension, 400 mg/ 5 mL based on data shown in Table 1 A. As shown, no less than 80% of the labeled amount of Eslicarbazepine Acetate is dissolved in 30 minutes. For reference, dissolution data for commercially available APTIOM (Eslicarbazepine Acetate) Tablets 800 mg, manufactured by Sunovion, are provided in Table 1B below.

**Table 1A: Dissolution profile of Eslicarbazepine Acetate Oral Suspension**

| **Ref.** | **Collection Times (minutes) Eslicarbazepine Acetate** | | | | | |
|---|---|---|---|---|---|---|
| | **5** | **10** | **15** | **20** | **30** | **45** |
| 1 | 77.5 | 89.9 | 94.5 | 96.0 | 98.2 | 98.6 |
| 2 | 86.5 | 92.9 | 96.9 | 96.2 | 98.2 | 97.8 |
| 3 | 45.9 | 63.2 | 67.6 | 69.9 | 73.5 | 76.5 |
| 4 | 62.7 | 70.6 | 76.3 | 79.9 | 83.0 | 87.8 |
| 5 | 65.5 | 79.6 | 82.2 | 87.2 | 88.6 | 90.3 |
| 6 | 79.4 | 89.8 | 93.9 | 95.6 | 97.4 | 97.9 |
| 7 | 74.7 | 86.0 | 92.6 | 95.2 | 98.0 | 96.2 |
| 8 | 62.6 | 79.1 | 85.7 | 89.5 | 94.1 | 94.7 |
| 9 | 59.6 | 76.9 | 83.8 | 87.9 | 91.6 | 92.5 |
| 10 | 76.8 | 88.7 | 93.0 | 94.9 | 96.4 | 94.5 |
| 11 | 57.2 | 69.8 | 73.3 | 77.0 | 81.5 | 83.2 |
| 12 | 73.0 | 83.3 | 85.4 | 88.7 | 90.7 | 91.7 |
| Mean | 77.5 | 89.9 | 94.5 | 96.0 | 98.2 | 98.6 |
| Range | 46-87 | 63-93 | 68-97 | 70-96 | 74-98 | 77-99 |
| % RSD | 17.52 | 11.85 | 10.99 | 9.78 | 8.98 | 7.26 |

**Table 1B: Dissolution profile of APTIOM (Eslicarbazepine Acetate) Tablets 800 mg**

| **Ref.** | **Collection Times (minutes) Eslicarbazepine Acetate** | | | | | |
|---|---|---|---|---|---|---|
| | **5** | **10** | **15** | **20** | **30** | **45** |
| 1 | 35.8 | 64.7 | 76.3 | 80.1 | 84.5 | 86.4 |
| 2 | 35.1 | 64.4 | 76.6 | 80.3 | 85.1 | 87.5 |
| 3 | 35.1 | 63.6 | 76.5 | 80.6 | 84.8 | 87.3 |
| 4 | 36.6 | 65.5 | 76.4 | 80.8 | 84.0 | 85.6 |
| 5 | 36.5 | 64.5 | 76.3 | 80.6 | 84.1 | 85.9 |
| 6 | 37.0 | 64.8 | 76.2 | 80.5 | 84.1 | 85.8 |
| 7 | 33.6 | 63.1 | 76.6 | 80.4 | 84.1 | 86.5 |
| 8 | 35.5 | 64.6 | 76.8 | 81.0 | 84.6 | 87.0 |
| 9 | 36.4 | 65.2 | 76.4 | 80.0 | 83.4 | 83.7 |
| 10 | 36.5 | 64.9 | 75.9 | 79.8 | 83.0 | 84.1 |
| 11 | 36.0 | 65.2 | 76.3 | 80.1 | 83.9 | 86.2 |
| 12 | 37.2 | 65.7 | 76.5 | 80.2 | 83.9 | 85.7 |
| Mean | 36 | 65 | 76 | 80 | 84 | 86 |
| Range | 34-37 | 63-66 | 76-77 | 80-81 | 83-85 | 84-88 |
| % RSD | 2.79 | 1.15 | 0.30 | 0.44 | 0.69 | 1.34 |

### Stability studies

A study was conducted to determine the stability profile of an Eslicarbazepine Acetate Oral Suspension of Example 2. The prepared formulations were subjected to stability evaluation under accelerated conditions (40°C ± 2°C / 75% RH ± 5% RH) for up to 6 months, long-term storage conditions (25°C ± 2°C / 60% RH ± 5% RH) for up to 6 months, and intermediate conditions (30°C ± 2°C / 75% RH ± 5% RH) for up to 6 months. The stability data are presented in Table 2 below.

**Table 2: Stability of Eslicarbazepine Acetate Oral Suspension of Example 2**

| **Period** | | **% Impurities** | | | | **% Assay** |
|---|---|---|---|---|---|---|
| **Specifications** | **Eslicarbazepine NMT 0.5%** | **Oxcarbazepine NMT 0.15** | **Carbamazepine NMT 0.15** | **Any Unspecified Degradation product NMT 0.10** | **Total IMP. NMT 2.0%** | **90.0-110.0** |
| **Initial** | ND | ND | ND | ND | ND | 103.3 |
| **Long term-3 month** | ND | ND | ND | 0.04 | 0.04 | 102.8 |
| **Accelerated-3 month** | 0.08 | ND | ND | 0.07 | 0.15 | 102.2 |
| **Long term-6 month** | ND | ND | ND | 0.06 | 0.1 | 102.2 |
| **Accelerated-6 month** | 0.2 | ND | ND | 0.08 | 0.3 | 101.1 |
| **Intermediate-6 month** | ND | ND | ND | 0.07 | 0.1 | 100.8 |

The addition of Carbomer and Microcrystalline Cellulose in present formulations, combined with PEG stearate, appears to enhance the stability of the suspension across 3.0-6.5 pH levels, evidenced by lower impurity percentages.

### Pharmacokinetic studies

The pharmacokinetic (PK) properties of Eslicarbazepine suspension of Example 1 (T1), Example 2 (T2), and the Aptiom^{®} (eslicarbazepine acetate) tablets 800mg as Reference (R) were evaluated. These evaluations were conducted as part of a randomized crossover study under fasting conditions with a 7-day washout period. The key PK parameters assessed included the maximum observed plasma concentration (Cmax), area under the plasma concentration-time curve from time zero to the last quantifiable concentration (AUC₀₋ₜ), area under the plasma concentration-time curve extrapolated to infinity (AUC_{0-∞}), time to reach maximum concentration (Tmax), elimination half-life (t₁/₂), and elimination rate constant (Kel). **Table 3** summarizes the mean values and standard deviations (SD) for each parameter, providing a comparative analysis of the PK profiles of the tested formulations. **Figure 2** is a graph of Mean Plasma Eslicarbazepine Concentrations versus Time on a linear scale. It compares the plasma concentration profiles for Treatment 1 (T1), Treatment 2 (T2), and the Reference (R) over time.

Additionally, the pharmacokinetic parameters (Cmax, AUC₀₋ₜ, and AUC_{0-∞}) demonstrated geometric mean ratios with 90% confidence intervals within the bioequivalence range of 80-125%. The results confirmed comparable bioavailability and tolerability to Aptiom^{®} Eslicarbazepine Acetate Tablets, supporting the suitability of the test formulations for therapeutic use.

**Table 3: Mean (SD) of Pharmacokinetic Parameters of Eslicarbazepine**

| **PK Parameter (Units)** | **Treatment Example 1 (T1)** | **Treatment Example 2 (T2)** | **Treatment R (Reference)** |
|---|---|---|---|
| Cₘₐₓ (ng/mL) | 13304.426 (1876.406) | 16659.736 (3182.433) | 14550.375 (1956.923) |
| AUC₀₋ₜ (ng.hr/mL) | 306218.705 (45699.578) | 306241.176 (47857.185) | 269894.455 (36766.646) |
| AUC_{0-∞} (ng.hr/mL) | 324493.759 (45615.301) | 321817.824 (47460.185) | 288984.991 (41192.931) |
| Tₘₐₓ (hr.) | 4.5 (2.25, 6) | 2 (1.25, 4.5) | 1.89 (1, 3.67) |
| t_{1/2} (hr.) | 11.137 (1.278) | 10.858 (1.635) | 11.78 (1.685) |
| Kel (1/hr.) | 0.063 (0.007) | 0.065 (0.01) | 0.06 (0.009) |

### Statistical Results

**Table 4a: Statistical Results of Assessment of bioavailability of Eslicarbazepine under fasting conditions Eslicarbazepine acetate oral suspension 400mg/5mL (10mL eq. to800mg) (Example 1) with Aptiom^{®} (eslicarbazepine acetate) tablets 800mg (Form R) (N = 10)**

| **PK Parameter** | **Least Squares Geometric Means of Treatment** | | **Ratio (%)** | **Intra-subject %CV** | **90% CI of Ratio** |
|---|---|---|---|---|---|
| | **Example 1 (T1)** | **Reference (R)** | | | |
| log (Cₘₐₓ) (ng/mL) | 13189.21 | 14389.60 | 91.66 | 7.1 | 86.73, 96.87 |
| log (AUC₀₋ₜ) (hng/mL) | 302024.44 | 266199.13 | 113.46 | 4.0 | 109.91, 117.12 |
| log (AUC_{0-∞}) (hng/mL) | 320981.06 | 284972.37 | 112.64 | 3.4 | 109.63, 115.72 |

**Table 4b: Statistical Results of Assessment of bioavailability of Eslicarbazepine under fasting conditions Eslicarbazepine acetate oral suspension 400mg/5mL (10mL eq. to 800mg) (Example 2) with Aptiom^{®} (eslicarbazepine acetate) tablets 800mg (Form R) (N = 10)**

| **PK Parameter** | **Least Squares Geometric Means of Treatment** | | **Ratio (%)** | **Intra-subject %CV** | **90% CI of Ratio** |
|---|---|---|---|---|---|
| | **Example 2 (T2)** | **Reference (R)** | | | |
| log (Cₘₐₓ) (ng/mL) | 16350.26 | 14389.60 | 113.63 | 7.1 | 107.51, 120.09 |
| log (AUC₀₋ₜ) (hng/mL) | 302082.59 | 266199.13 | 113.48 | 4.0 | 109.93, 117.14 |
| log (AUC_{0-∞}) (hng/mL) | 318091.63 | 284972.37 | 111.62 | 3.4 | 108.65, 114.68 |

The study evaluated the bioavailability of two Eslicarbazepine acetate oral suspension formulations (Example 1 and Example 2, 400 mg/5 mL, 10 mL = 800 mg) compared to Aptiom^{®} 800 mg tablets in 12 healthy adult participants under fasting conditions. This was conducted using a randomized, three-way crossover design with a 7-day washout period. The bioavailability results are set forth in Tables 4a and 4b. The geometric mean (GM) ratio and 90% confidence intervals (CIs) for Example 1 vs. Aptiom^{®} were as follows: Cmax 91.66 (86.73, 96.87), AUC0-t 113.46 (109.91, 117.12), AUC0-∞ 112.64 (109.63, 115.72). For Example, 2 vs. Aptiom^{®}, the values were: Cmax 113.63 (107.51, 120.09), AUC0-t 113.48 (109.93, 117.14), AUC0-∞ 111.62 (108.65, 114.68). All CIs were within the bioequivalence range of 80-125%. Both test formulations were found to be bioequivalent to Aptiom^{®}, and all treatments were well tolerated.

The present invention provides a stable, immediate-release liquid suspension of Eslicarbazepine Acetate for the treatment of partial-onset seizures. This formulation demonstrates rapid dissolution, stability under various conditions, and bioequivalence with existing tablet formulations, making it a viable alternative for patients requiring liquid dosage forms. This innovation meets the need for patient-friendly formulations without compromising therapeutic efficacy.

### ADDITIONAL DISCLOSURE

The following are non-limiting, specific embodiments in accordance with the present disclosure:
A first embodiment, which is an immediate-release, liquid oral pharmaceutical suspension dosage form comprising: (a) Eslicarbazepine Acetate, or a pharmaceutically acceptable salt, ester, hydrate, or polymorph thereof, in an amount ranging from about 5 to about 10 g per 100 mL of the suspension, (b) carbomer, in an amount ranging from about 0.01 g to about 1.0 g per 100 mL of the suspension, (c) Microcrystalline cellulose, in an amount ranging from about 0.1 g to about 3.0 g per 100 mL of the suspension, (d) optionally Polyethylene Glycol 8 Stearate, an antimicrobial agent, a sweetening agent, a flavoring agent, or any combination thereof, (e) Purified water, as the carrier, q.s. to 100 mL, and (f) one or more buffers or pH adjusting agents, present in an amount effective to provide the suspension with a pH of from about 3.0 to about 6.5.

A second embodiment, which is an immediate-release, liquid oral pharmaceutical suspension dosage form of the first embodiment, wherein the antimicrobial agent comprises at least one paraben or a mixture of parabens in a concentration ranging from about 0.01% to 0.3% w/v.

A third embodiment, which is an immediate-release, liquid oral pharmaceutical suspension dosage form of the first or second embodiment, wherein the sweetening agent is selected from sugars, sugar alcohols, sugar substitutes, or any combination thereof in an amount ranging from about 0.1% to 20% w/v.

A fourth embodiment, which is an immediate-release, liquid oral pharmaceutical suspension dosage form of the first or second embodiment, wherein the sweetening agent is selected from sucrose, dextrose, lactose, sorbitol, mannitol, xylitol, sucralose, saccharin, aspartame, or any combination thereof in an amount ranging from about 0.1% to 20% w/v.

A fifth embodiment, which is an immediate-release, liquid oral pharmaceutical suspension dosage form of any of the first to fourth embodiments, wherein a flavoring agent is selected from fruit essences, synthetic flavors, natural oils, aromatics, or any combination thereof, in an amount ranging from 0.01% to 5% w/v.

A sixth embodiment, which is an immediate-release, liquid oral pharmaceutical suspension dosage form of any of the first to fourth embodiments, wherein a flavoring agent is selected from apple essence, banana essence, strawberry essence, orange essence, grape essence, synthetic vanilla, synthetic bubble gum, synthetic peppermint, cinnamon oil, peppermint oil, clove oil, citrus oil, maltol, menthol, ethyl vanillin, or any combination thereof, in an amount ranging from 0.01% to 5% w/v.

A seventh embodiment, which is an immediate-release, liquid oral pharmaceutical suspension dosage form of any of the first to sixth embodiments, wherein it further comprises a surfactant selected from non-ionic surfactants, anionic surfactants, cationic surfactants, zwitterionic surfactants, or any combination thereof in an amount ranging from about 0.1% to 3% w/v.

An eighth embodiment, which is an immediate-release, liquid oral pharmaceutical suspension dosage form of any of the first to seventh embodiments, further comprising an antioxidant selected from ascorbic acid, tert-butylhydroquinone, sodium pyrosulfite, tocopherol, and others in an amount up to about 10% w/v.

A ninth embodiment, which is the pharmaceutical composition of any of the first to eighth embodiments, further comprising a bulking agent selected from sucrose, sugar alcohols, starch, calcium carbonate, calcium phosphate, cellulose, maltodextrin, lactose, or combination thereof in a concentration of about 0.1% to 20% w/v.

A tenth embodiment, which is the pharmaceutical composition of the ninth embodiment, wherein sugar alcohols are selected from glycerin, sorbitol, xylitol, erythritol, or combination thereof in a concentration of about 0.1% to 20% w/v.

An eleventh embodiment, which is an immediate-release, liquid oral pharmaceutical suspension dosage form comprising: (a) Eslicarbazepine Acetate, or a pharmaceutically acceptable salt, ester, hydrate, or polymorph thereof, in an amount from about 5 to about 10 g per 100 mL of the suspension, (b) carbomer, in an amount ranging from about 0.01 g to about 1.0 g per 100 mL of the suspension, (c) Microcrystalline cellulose, in an amount ranging from about 0.1 g to about 3.0 g per 100 mL of the suspension, (d) Polyethylene Glycol 8 Stearate, in an amount ranging from about 0.01 g to about 3.0 g per 100 mL of the suspension, (e) at least one paraben preservative in an amount ranging from about 0.01 g to about 0.30 g per 100 mL of the suspension, (f) Sucralose, NF in an amount ranging from about 0.10 g to about 1.0 g per 100 mL of the suspension, (g) a flavoring agent in an amount ranging from about 0.1 g to about 1.0 g per 100 mL of the suspension, (h) Purified water, as the carrier, q.s. to 100 mL; an, (i) one or more buffers or pH adjusting agents selected from citrate buffers, phosphate buffers, or sodium hydroxide, present in an amount effective to provide the suspension with a pH of from about 3.0 to about 6.5.

A twelfth embodiment, which is an immediate-release, liquid oral pharmaceutical suspension dosage form comprising: (a) Eslicarbazepine Acetate, or a pharmaceutically acceptable salt, ester, hydrate, or polymorph thereof, in an amount of about 8.0 g per 100 mL of the suspension; (b) carbomer, in an amount from about 0.05 g to about 0.15 g per 100 mL, (c) Microcrystalline cellulose, in an amount from about 0.3 g to about 1.0 g per 100 mL, (d) Polyethylene Glycol 8 Stearate, in an amount of about 0.1 g per 100 mL, (e) Methylparaben, in an amount of about 0.12 g per 100 mL, (f) Propylparaben, in an amount of about 0.03 g per 100 mL, (g) Sucralose, in an amount of about 0.30 g per 100 mL, (h) Natural Peppermint Flavor, in an amount of about 0.30 g per 100 mL, (i) Sodium hydroxide for pH adjustment; and, (j) Purified water, as the carrier, q.s. to 100 mL, wherein the pH of the suspension is from about 4.4 to about 5.4.

A thirteenth embodiment, which is the immediate-release oral pharmaceutical suspension of any of the first to twelfth embodiments, wherein the suspension has long-term stability with less than 2% total impurities of the eslicarbazepine content.

A fourteenth embodiment, which is the immediate-release oral pharmaceutical suspension of any of the first to thirteenth embodiments, wherein the viscosity of the suspension is from 30 cps to 850 cps as measured by a Brookfield viscometer using spindle LV-3 at 100 rpm and 25°C.

A fifteenth embodiment, which is the immediate-release oral pharmaceutical suspension of any of the first to fourteenth embodiments, wherein the viscosity is tailored to range from 100 cps to 300 cps for ease of administration to paediatric patients.

A sixteenth embodiment, which is the immediate-release oral pharmaceutical suspension of any of the first to fifteenth embodiments, wherein the Eslicarbazepine Acetate has a D90 particle size less than 250 µm and D50 particle size less than 150 µm.

A seventeenth embodiment, which is the immediate-release oral pharmaceutical suspension any of the first to sixteenth embodiments, wherein the suspension exhibits more than 75% of drug release within 15 minutes when placed in a dissolution vessel filled with 1000 mL of acetate buffer, pH 4.5, maintained at 37 ± 0.5°C and stirred at a paddle speed of 100 rpm using a USP Type II (paddle) apparatus.

An eighteenth embodiment, which is the immediate-release oral pharmaceutical suspension of any of the first to seventeenth embodiments, wherein the suspension is bioequivalent to marketed Eslicarbazepine Acetate Oral Tablets (200 mg, 400 mg, 600 mg, and 800 mg) based on pharmacokinetic parameters (Cmax, AUC0-t, and AUC0-∞) with geometric mean ratios and 90% confidence intervals within the bioequivalence range of 80-125%.

A nineteenth embodiment, which is the immediate-release oral pharmaceutical suspension of any of the first to eighteenth embodiments, wherein the pH of the suspension is about 4.9, and after storage at 60°C for 1-week, unknown impurities are less than 0.05% of the total eslicarbazepine content.

A twentieth embodiment, which is the immediate-release oral pharmaceutical suspension of any of the first to eighteenth embodiments, wherein the pH of the suspension is about 5.4, and after storage at 60°C for 1-week unknown impurities are less than 0.03% of the total eslicarbazepine content.

A twenty-first embodiment, which is the immediate-release oral pharmaceutical suspension of any of the first to twentieth embodiments, wherein the suspension maintains chemical stability and exhibits less than 0.5% degradation of Eslicarbazepine Acetate after storage at 40°C and 75% relative humidity for six months.

A twenty-second embodiment, which is a method of preparing an immediate-release oral pharmaceutical suspension dosage form, comprising: (a) Preparing a preservative phase (A) by: Heating purified water to a temperature of 80-85°C; Adding methylparaben and propylparaben to the heated water and mixing until dissolved; Cooling the mixture to 40-45°C and dispersing carbomer homopolymer and microcrystalline cellulose into the mixture; Adding buffering agents and sweeteners to the mixture and further cooling to 25-30°C, (b) Preparing an API phase (B) by: Heating 0.5 to 50 g of purified water to a temperature of 45-55°C; Mixing polyethylene glycol 400 monostearate into the heated water and cooling the mixture to 25-30°C; Adding Eslicarbazepine Acetate to form a slurry, (c) Combining the API phase (B) with the preservative phase (A), wherein phase (B) is added to phase (A) at a temperature of 25-30°C, (d) Adjusting the pH of the combined mixture (A and B) to between about 4.4 and 5.4 using sodium hydroxide, and (e) Vigorously mixing the resulting combined mixture to form a homogeneous suspension.

A twenty-third embodiment, which is a method of treating partial onset seizures in a patient in need thereof, comprising administering to the patient the immediate-release, liquid oral pharmaceutical suspension dosage form of any of the first to twenty-first embodiments.

Accordingly, the scope of protection is not limited by the description set out above but is only limited by the claims which follow, that scope including all equivalents of the subject matter of the claims. Each and every claim is incorporated into the specification as embodiments of the present disclosure. Thus, the claims are a further description and are an addition to the embodiments of the present disclosure. The discussion of a reference herein is not an admission that it is prior art, especially any reference that can have a publication date after the priority date of this application. The disclosures of all patents, patent applications, and publications cited herein are hereby incorporated by reference, to the extent that they provide exemplary, procedural, or other details supplementary to those set forth herein.

While several embodiments have been provided in the present disclosure, it should be understood that the disclosed systems and methods may be embodied in many other specific forms without departing from the spirit or scope of the present disclosure. The present examples are to be considered as illustrative and not restrictive, and the intention is not to be limited to the details given herein. For example, the various elements or components may be combined or integrated in another system or certain features may be omitted or not implemented.

Also, techniques, systems, subsystems, and methods described and illustrated in the various embodiments as discrete or separate may be combined or integrated with other systems, modules, techniques, or methods without departing from the scope of the present disclosure. Other items shown or discussed as directly coupled or communicating with each other may be indirectly coupled or communicating through some interface, device, or intermediate component, whether electrically, mechanically, or otherwise. Other examples of changes, substitutions, and alterations are ascertainable by one skilled in the art and could be made without departing from the spirit and scope disclosed herein.

## Claims

1. An immediate-release, liquid oral pharmaceutical suspension dosage form comprising:
(a) eslicarbazepine acetate, or a pharmaceutically acceptable salt, ester, hydrate, or polymorph thereof, in an amount ranging from about 5 to about 10 g per 100 mL of the suspension;
(b) carbomer, in an amount ranging from about 0.01 g to about 1.0 g per 100 mL of the suspension;
(c) microcrystalline cellulose, in an amount ranging from about 0.1 g to about 3.0 g per 100 mL of the suspension;
(d) optionally polyethylene glycol 8 stearate, an antimicrobial agent, a sweetening agent, a flavoring agent, or any combination thereof;
(e) purified water, as the carrier, q.s. to 100 mL; and
(f) one or more buffers or pH adjusting agents, present in an amount effective to provide the suspension with a pH of from about 3.0 to about 6.5.

2. An immediate-release, liquid oral pharmaceutical suspension dosage form of claim 1, wherein the antimicrobial agent comprises at least one paraben or a mixture of parabens in a concentration ranging from about 0.01% to 0.3% w/v., preferably the sweetening agent is selected from sugars, sugar alcohols, sugar substitutes, or any combination thereof in an amount ranging from about 0.1% to 20% w/v, more preferably the sweetening agent is selected from sucrose, dextrose, lactose, sorbitol, mannitol, xylitol, sucralose, saccharin, aspartame, or any combination thereof in an amount ranging from about 0.1% to 20% w/v.

3. An immediate-release, liquid oral pharmaceutical suspension dosage form of claim 1 or 2, wherein a flavoring agent is selected from fruit essences, synthetic flavors, natural oils, aromatics, or any combination thereof, in an amount ranging from 0.01% to 5% w/v, preferably the flavoring agent is selected from apple essence, banana essence, strawberry essence, orange essence, grape essence, synthetic vanilla, synthetic bubble gum, synthetic peppermint, cinnamon oil, peppermint oil, clove oil, citrus oil, maltol, menthol, ethyl vanillin, or any combination thereof, in an amount ranging from 0.01% to 5% w/v.

4. An immediate-release, liquid oral pharmaceutical suspension dosage form of any of claims 1-3, wherein it further comprises a surfactant selected from non-ionic surfactants, anionic surfactants, cationic surfactants, zwitterionic surfactants, or any combination thereof in an amount ranging from about 0.1% to 3% w/v, or an antioxidant selected from ascorbic acid, tert-butylhydroquinone, sodium pyrosulfite, tocopherol, and others in an amount up to about 10% w/v.

5. The pharmaceutical composition of any of claims 1-4, further comprising a bulking agent selected from sucrose, sugar alcohols, starch, calcium carbonate, calcium phosphate, cellulose, maltodextrin, lactose, or combination thereof in a concentration of about 0.1% to 20% w/v, preferably sugar alcohols selected from glycerin, sorbitol, xylitol, erythritol, or combination thereof in a concentration of about 0.1% to 20% w/v.

6. An immediate-release, liquid oral pharmaceutical suspension dosage form comprising:
(a) eslicarbazepine acetate, or a pharmaceutically acceptable salt, ester, hydrate, or polymorph thereof, in an amount from about 5 to about 10 g per 100 mL of the suspension;
(b) carbomer, in an amount ranging from about 0.01 g to about 1.0 g per 100 mL of the suspension;
(c) microcrystalline cellulose, in an amount ranging from about 0.1 g to about 3.0 g per 100 mL of the suspension;
(d) polyethylene glycol 8 stearate, in an amount ranging from about 0.01 g to about 3.0 g per 100 mL of the suspension;
(e) at least one paraben preservative in an amount ranging from about 0.01 g to about 0.30 g per 100 mL of the suspension;
(f) sucralose, in an amount ranging from about 0.10 g to about 1.0 g per 100 mL of the suspension;
(g) a flavoring agent in an amount ranging from about 0.1 g to about 1.0 g per 100 mL of the suspension;
(h) purified water, as the carrier, q.s. to 100 mL; and
(i) one or more buffers or pH adjusting agents selected from citrate buffers, phosphate buffers, or sodium hydroxide, present in an amount effective to provide the suspension with a pH of from about 3.0 to about 6.5.

7. An immediate-release, liquid oral pharmaceutical suspension dosage form comprising:
(a) eslicarbazepine acetate, or a pharmaceutically acceptable salt, ester, hydrate, or polymorph thereof, in an amount of about 8.0 g per 100 mL of the suspension;
(b) carbomer, in an amount from about 0.05 g to about 0.15 g per 100 mL;
(c) microcrystalline cellulose, in an amount from about 0.3 g to about 1.0 g per 100 mL;
(d) polyethylene glycol 8 stearate, in an amount of about 0.1 g per 100 mL;
(e) methylparaben, in an amount of about 0.12 g per 100 mL;
(f) propylparaben, in an amount of about 0.03 g per 100 mL;
(g) sucralose, in an amount of about 0.30 g per 100 mL;
(h) natural peppermint flavor, in an amount of about 0.30 g per 100 mL;
(i) sodium hydroxide for pH adjustment; and
(j) purified water, as the carrier, q.s. to 100 mL;
wherein the pH of the suspension is from about 4.4 to about 5.4.

8. The immediate-release oral pharmaceutical suspension of any of claims 1-7, wherein the suspension has long-term stability with less than 2% total impurities of the eslicarbazepine content.

9. The immediate-release oral pharmaceutical suspension of any of claims 1-8, wherein the viscosity of the suspension is from 30 cps to 850 cps as measured by a Brookfield viscometer using spindle LV-3 at 100 rpm and 25°C, preferably the viscosity is tailored to range from 100 cps to 300 cps for ease of administration to paediatric patients.

10. The immediate-release oral pharmaceutical suspension of any of claims 1-9, wherein the eslicarbazepine acetate has a D90 particle size less than 250 µm and D50 particle size less than 150 µm.

11. The immediate-release oral pharmaceutical suspension of any of claims 1-10, wherein the suspension exhibits more than 75% of drug release within 15 minutes when placed in a dissolution vessel filled with 1000 mL of acetate buffer, pH 4.5, maintained at 37 ± 0.5°C and stirred at a paddle speed of 100 rpm using a USP Type II (paddle) apparatus.

12. The immediate-release oral pharmaceutical suspension of any of claims 1-11, wherein the suspension is bioequivalent to marketed eslicarbazepine acetate oral tablets (200 mg, 400 mg, 600 mg, and 800 mg) based on pharmacokinetic parameters (Cmax, AUC0-t, and AUC0-∞) with geometric mean ratios and 90% confidence intervals within the bioequivalence range of 80-125%.

13. The immediate-release oral pharmaceutical suspension of any of claims 1-12, wherein the pH of the suspension is about 4.9, and after storage at 60°C for 1-week, unknown impurities are less than 0.05% of the total eslicarbazepine content, preferagbly the pH of the suspension is about 5.4, and after storage at 60°C for 1-week unknown impurities are less than 0.03% of the total eslicarbazepine content.

14. The immediate-release oral pharmaceutical suspension of any of claims 1-14, wherein the suspension maintains chemical stability and exhibits less than 0.5% degradation of eslicarbazepine acetate after storage at 40°C and 75% relative humidity for six months.

15. A method of preparing an immediate-release oral pharmaceutical suspension dosage form of any of claims 1-14, comprising:
(a) preparing a preservative phase (A) by: heating purified water to a temperature of 80-85°C; adding methylparaben and propylparaben to the heated water and mixing until dissolved; cooling the mixture to 40-45°C and dispersing carbomer homopolymer and microcrystalline cellulose into the mixture; adding buffering agents and sweeteners to the mixture and further cooling to 25-30°C;
(b) preparing an API phase (B) by: heating 0.5 to 50 g of purified water to a temperature of 45-55°C; mixing polyethylene glycol 400 monostearate into the heated water and cooling the mixture to 25-30°C; adding eslicarbazepine acetate to form a slurry;
(c) combining the API phase (B) with the preservative phase (A), wherein phase (B) is added to phase (A) at a temperature of 25-30°C;
(d) adjusting the pH of the combined mixture (A and B) to between about 4.4 and 5.4 using sodium hydroxide; and
(e) vigorously mixing the resulting combined mixture to form a homogeneous suspension.
